(19) **Europäisches Patentamt**

**European Patent Office**

**Office européen des brevets**

(11) **EP 0 721 354 B1**

(12) **EUROPÄISCHE PATENTSCHRIFT**

(45) Veröffentlichungstag und Bekanntmachung des Hinweises auf die Patenterteilung:
**02.02.2000 Patentblatt 2000/05**

(21) Anmeldenummer: **94928386.5**

(22) Anmeldetag: **21.09.1994**

(51) Int. Cl.⁷: $A61L\ 15/00$, $C08F\ 8/00$

(86) Internationale Anmeldenummer:
**PCT/EP94/03153**

(87) Internationale Veröffentlichungsnummer:
**WO 95/09014 (06.04.1995 Gazette 1995/15)**

(54) **PULVERFÖRMIGE, WÄSSRIGE FLÜSSIGKEITEN ABSORBIERENDE POLYMERE, VERFAHREN ZU IHRER HERSTELLUNG UND IHRE VERWENDUNG ALS ABSORPTIONSMITTEL**

POWDER-FORM POLYMERS CAPABLE OF ABSORBING AQUEOUS LIQUIDS, METHOD OF PREPARING THEM AND THEIR USE AS ABSORBENTS

POLYMERES PULVERULENTS ABSORBANT DES LIQUIDES AQUEUX, LEUR PROCEDE DE PREPARATION ET LEUR UTILISATION COMME ABSORBANTS

(84) Benannte Vertragsstaaten:
**AT BE CH DE DK ES FR GB GR IE IT LI LU MC NL PT SE**

(30) Priorität: **29.09.1993 DE 4333056**

(43) Veröffentlichungstag der Anmeldung:
**17.07.1996 Patentblatt 1996/29**

(73) Patentinhaber:
**Stockhausen GmbH & Co. KG**
**47805 Krefeld (DE)**

(72) Erfinder:
• **BREHM, Helmut**
  **D-47800 Krefeld (DE)**
• **HARTAN, Hans-Georg**
  **D-47625 Kevelaer (DE)**

(74) Vertreter:
**Wolff, Felix, Dr. et al**
**Kutzenberger & Wolff**
**Theodor-Heuss-Ring 23**
**50668 Köln (DE)**

(56) Entgegenhaltungen:
**EP-A- 0 031 628      EP-A- 0 317 106**
**FR-A- 2 559 158**

## Beschreibung

Die Erfindung betrifft pulverförmige vernetzte Wasser und wäßrige Flüssigkeiten absorbierende, als Superabsorber bezeichnete Polymere mit verbesserten Quellungseigenschaften und verbessertem Rückhaltevermögen für wäßrige Flüssigkeiten, ein Verfahren zur Herstellung dieser Polymeren sowie ihre Verwendung in absorbierenden Sanitärartikeln und in technischen Bereichen.

Superabsorber sind wasserunlösliche, vernetzte Polymere, die in der Lage sind, unter Quellung und Ausbildung von Hydrogelen große Mengen an Körperflüssigkeiten, wie Urin oder Blut oder andere wäßrige Flüssigkeiten aufzunehmen und die absorbierte Flüssigkeitsmenge unter einem bestimmten Druck zurückzuhalten. Wegen dieser charakteristischen Absorptionseigenschaften finden die Polymeren hauptsächlich Anwendung in Sanitärartikeln, z. B. in Babywindeln und Damenbinden.

Bei den gegenwärtig kommerziell verfügbaren Superabsorbern handelt es sich um vernette Polyacrylsäuren oder vernetzte Stärke-Acrylsäure-Pfropfpolymerisate, die teilweise mit Natron- oder Kalilauge neutralisiert sind. Die Herstellung von pulverförmigen Superabsorbern erfolgt prinzipiell nach zwei Methoden:

Nach der ersten Methode wird teilneutralisierte Acrylsäure in wäßriger Lösung in Gegenwart eines mehrfunktionellen Vernetzers durch radikalische Polymerisation in ein Gel überführt, das dann zerkleinert, getrocknet, gemahlen und aufdie gewünschte Partikelgröße abgesiebt wird. Die Lösungspolymerisation kann kontinuierlich oder diskontinuierlich durchgeführt werden. Die Patentliteratur weist ein breites Spektrum an Variationsmöglichkeiten hinsichtlich der Konzentrationsverhältnisse, Temperaturen, Art und Menge der Vernetzer und Initiatoren aus. Typische Verfahren sind z. B. in den Patenten US 4 286 082, DE 27 06 135 und US 4 076 663 beschrieben.

Die zweite Methode umfaßt das inverse Suspensions- und Emulsionspolymerisationsverfahren. In diesen Prozessen wird eine wäßrige, teilneutralisierte Acrylsäurelösung mit Hilfe von Schutzkolloiden oder Emulgatoren in einem hydrophoben, organischen Lösungsmittel dispergiert und durch Radikalinitiatoren die Polymerisation gestartet. Nach Beendigung der Polymerisation wird das Wasser aus dem Reaktionsgemisch azeotrop entfernt, das Polymerprodukt abfiltriert und getrocknet. Die Vernetzungsreaktion kann durch Einpolymerisieren eines in der Monomerenlösung gelösten polyfunktionellen Vernetzers und/oder durch Reaktion geeigneter Vernetzungsmittel mit funktionellen Gruppen des Polymeren während einer der Herstellungsschritte erfolgen. Das Verfahrensprinzip ist beispielsweise in den Patenten US 43 40 706, DE 37 13 601 und DE 28 40 010 beschrieben.

Während bei der Entwicklung der Superabsorber zunächst allein das sehr hohe Quellvermögen bei Kontakt des Absorbers mit der Flüssigkeit, auch als freie Quellkapazität bezeichnet, im Vordergrund stand, hat sich später gezeigt, daß es nicht nur auf die Menge der absorbierten Flüssigkeit, sondern auch auf die Festigkeit des gequollenen Gels ankommt. Das Absorptionsvermögen, Quellvermögen oder freie Quellkapazität genannt, einerseits, und die Gelfestigkeit andererseits, stellen jedoch gegenläufige Eigenschaften dar, wie aus US 32 47 171 und US Re 32 649 bekannt ist. Das bedeutet, daß Polymere mit besonders hohem Absorptionsvermögen nur eine geringe Festigkeit des gequollenen Gels aufweisen, mit der Folge, daß das Gel unter einem angewendeten Druck, bei Belastung durch das Körpergewicht deformierbar ist und gleichzeitig die weitere Flüssigkeitsverteilung und Flüssigkeitsaufnahme verhindert wird. Nach der US Re 32 649 soll daher ein ausgewogenes Verhältnis der Eigenschaften derartiger Super-absorber in einer Windelkonstruktion zwischen Flüssigkeitsaufnahme, Flüssigkeitstransport und Trockenheit der Windel auf der Haut gewährleistet sein. Dabei kommt es nicht nur darauf an, daß das Polymere, nachdem es zunächst frei quellen konnte, die Flüssigkeit unter nachfolgender Einwirkung eines Drucks zurückhalten kann, sondern auch darauf, Flüssigkeiten auch gegen einen gleichzeitig wirkenden, d.h. während der Flüssigkeitsabsorption, ausgeübten Druck aufzunehmen, wie es unter praktischen Gegebenheiten geschieht, wenn eine Person auf einem Sanitärartikel sitzt, liegt, oder wenn es beispielsweise durch Beinbewegung zur Einwirkung von Scherkräften kommt. Diese spezifische Absorptionseigenschaft wird beispielsweise nach EP 0 339 461 A als Aufnahme unter Druck (AUL) bezeichnet.

Um superabsorbierende Polymeren bereitzustellen, welche die erforderliche Eigenschaftskombination, d. h. eine hohe Retentionskapazität, eine hohe Gelstärke und hohes Aufnahmevermögen unter Druck besitzen, ist es, wie bekannt, erforderlich, die pulverförmigen Polymeren nachträglich zu behandeln.

In der EP 0 083 022 B1, DE-OS 33 14 019 und DE 35 23 617 A1 wird eine oberflächenvernetztende Behandlung der Polymeren mit Verbindungen beschrieben, die mindestens zwei funktionelle mit Carboxylgruppen reaktionsfähige Gruppen enthalten, wobei insbesondere Polyole genannt werden.

Nach DE 40 20 780 C1 wird eine unter Druckbelastung verbesserte Quellfähigkeit eines superabsorbierenden Polymeren durch die Erwärmung des Polymerpulvers mit einem Alkylencarbonat, das gegebenenfalls mit Wasser und/oder Alkohol verdünnt aufgetra-gen wird, erreicht.

Durch die Nachbehandlung wasserquellbarer pulverförmiger Polymeren mit Verbindungen, die mit mehr als einer funktionellen Gruppe des Polymeren reagieren können, kommt es aber zwangsläufig gleichzeitig zu einer Abnahme der Quellkapazität. Die Abnahme der Quellkapazität durch die Nachbehandlung wird nach Angaben in EP 0 089 022 B1 und EP 0 450 923 A besonders hoch, wenn die Menge an Nachbehandlungsmittel zu groß gewählt wird.

Die Abnahme der Quellkapazität bzw. der Retention erfolgt demnach zwangsläufig, weil die Nachbehandlung eine zusätzliche Vernetzung der wasserquellbaren Polymer-partikel hervorruft.

Nach dem in DE 40 20 780 C1, EP 0 450 924 A und EP 0 339 461 A angegebenen Stand der Technik werden durch die Nachbehandlung wasserquellbarer, teilchenförmiger Polymeren wasserquellbarer Harze gewonnen und verwendet, die eine verbesserte Quellfähigkeit unter Druckbelastung zeigen, deren Retention mit Zunahme der Vernetzungsdichte und bei zunehmender Druckbelastung, beispielweise von 20 $g/cm^2$ auf 60 $g/cm^2$ von 26 g/g auf 8 g/g abnimmt. Der Sachverhalt ist zusammenfassend unter Berücksichtigung der Belegungsdichte in Tabelle 1 dargestellt.

Der aus verständlichen ästhetischen und unter wirtschaftlichen und ökologischen Aspekten zunehmenden Tendenz, die Sanitärartikel immer kleiner und dünner zu gestalten, kann vor allem dadurch entsprochen werden, daß man beispielsweise den großvolumigen Fluffanteil in Windeln reduziert und gleichzeitig den Anteil an Superabsorber erhöht. Hierbei muß der Superabsorber allerdings zusätzliche Aufgaben in Bezug auf Flüssigkeitsaufnahme und -transport übernehmen, die vorher vom Fluff erfüllt wurden.

Die Verwendung herkömmlicher Superabsorber in Windeln mit Anteilen von 40 oder 60 Gew% Superabsorber ist jedoch mit erheblichen Nachteilen verbunden, die den Einsatz

Tab.1

| | Behandlungsmittel | | Teabag Retention | Aufnahme unter Belastung bei | | | | |
|---|---|---|---|---|---|---|---|---|
| | Art | Menge | | 0 | 14 | 20 | 40 | 60g/cm$^2$ |
| | | % | g/g | g/g | | | | |
| DE 40 20 780 | | | | Belegungsdichte: 31,6 mg/cm$^2$ | | | | |
| Pulver A | - | - | 45$^{1)}$ | | | 6$^{2)}$ | | |
| Beisp. 9 | EC$^{4)}$ | 0,5 | 43 | | | 28 | | |
| 10 | EC | 1,0 | 41 | | | 32 | | |
| 11 | EC | 1,5 | 40 | | | 34 | | |
| 12 | EC | 2,0 | 37 | | | 34 | | |
| 13 | EC | 2,5 | 32 | | | 32 | | |
| EP A 04 50 924 | | | | Belegungsdichte: 5,2 mg/cm$^2$ | | | | |
| Pulver A1 | - | - | 54$^{2)}$ | | 10$^{3)}$ | | | |
| Beisp. 1 | GL$^{5)}$ | 0,75 | 42 | | 25 | | | |
| Pulver A2 | - | - | 62 | | 9,5 | | | |
| Beisp. 3 | GL | 1,0 | 43 | | 29 | | | |
| EP A 03 39 461 | | | | Belegungsdichte : 31,6 mg/cm$^2$ | | | | |
| Tab. B Beisp. 1 | keine Angabe | | | 42 | | 26$^{2)}$ | 13 | 8 |

$^{1)}$ 30 Min. abgeschleudert    0,9%ige NaCl
$^{2)}$ 60 Min        0,9%ige NaCl
$^{3)}$ 30 Min.        synth. Urin        1 psi = 6,895 · $10^3$ Pa
$^{4)}$ Ethylencarbonat
$^{5)}$ Glycerin

kommerziell erhältlicher Produkte stark einschränken. Als Ursache hierfür ist das bekannte Phenomen des "gel blokking", hervorgerufen durch koaguliertes Gel, und die damit verbundene Verringerung der Absorptionsgeschwindigkeit und -menge, insbesondere bei erhöhter Druckbelastung anzusehen.Es bestand daher die Aufgabe Polymerisate bereitzustellen, die als Superabsorber zur Verwendung in Windelkonstruktionen oder bei anderen technischen Anwendungen mit erhöhten Polymerisatanteilen bei erhöhter Flächen- oder Volumenbelegung eine hohe Quellkapazität unter Druckbelastung aufweisen.

Es wurde nun gefunden, daß wasserquellbare Polymere mit hoher Retention, erhöhter Quellkapazität unter Belastungen mit mehr als 20 $g/cm^2$ sowie auch hoher Quellkapazität unter Belastung bei erhöhter Belegungsdichte erhalten werden, wenn pulverförmige, wasserunlösliche, vernetzte, wäßrige oder seröse Flüssigkeiten wie Blut absorbierende Polymere, gebildet aus

a) 55 - 99,9 Gew.-% polymerisierten ungesättigten, polymerisierbaren Säuregruppen enthaltenden Monomeren, die zu mindestens 25 Mol-% neutralisiert sind,

b) 0 - 40 Gew.-% polymerisierten ungesättigten, mit a) copolymerisierbaren Monomeren,

c) 0,1 - 5 Gew.-% eines Vernetzungsmittels und

d) 0 - 30 Gew.-% eines wasserlöslichen Polymeren

wobei die Gewichtsmenge a) bis d) auf wasserfreies Polymer bezogen ist und die mit einer mit mindestens zwei funktionellen Gruppen der Polymeren, bevorzugt Säuregruppen, reaktionsfähigen Verbin-dung bei 150 °C - 250 °C oberflächenvernetzend behandelt wurden, nochmals mit 0,1 bis 5 Gew.-% einer, mit mindestens zwei funktionellen Gruppen der Polymeren reaktionsfähigen Verbindung in Gegenwart von 0,1 - 5 Gew.-% Wasser, bei Temperaturen von 150 °C-250 °C oberflächenvernetzend behandelt werden. Die Säuregruppen der Polymeren sind bevorzugt Carboxylgruppen.

Entgegen den Beobachtungen, daß bei zunehmender Vernetzungsdichte eine Verschlechterung der Absorptionseigenschaften eintritt, werden durch die erfindungsgemäße Verfahrensweise unter Wiederholung der oberflächenvernetzenden Behandlung Polymerisate erhalten, die überraschenderweise sowohl eine verbesserte Quellfähigkeit bei erhöhter Druckbelastung als auch bei erhöhter Flächenbelegung aufweisen.

Das erfindungsgemäß zu verwendende wasserabsorbierende Polymerisat wird erhalten durch Polymerisation von 55 - 99,9 Gew.-% Monomeren mit Säuregruppen, beispielsweise aus Acrylsäure, Methacrylsäure, 2-Acrylamido-2-methylpropansulfonsäure oder aus Mischungen dieser Monomeren; die Säuregruppen der Monomeren sind mindestens zu 25 Mol-% neutralisiert und liegen beispielsweise als Natrium-, Kalium- oder Ammoniumsalze vor. Vorzugsweise liegt der Neutralisationsgrad bei mindestens 50 Mol-%. Besonders bevorzugt ist ein Polymerisat, das aus vernetzter Acrylsäure oder Methacrylsäure gebildet ist, die zu 50 - 80 Mol-% neutralisiert ist.

Als weitere Monomeren können für die Herstellung der wasserabsorbierenden Polymerisate 0 bis 40 Gew.-% Acrylamid, Methacrylamid, Hydroxyethylacrylat, Dimethylaminoalkyl(meth)acrylat, Dimethylaminopropylacrylamid oder Acrylamidopropyltrimethylammoriumchlorid verwendet werden. Höhere Anteile als 40 % dieser Monomeren verschlechtern die Quellfähigkeit der Polymerisate.

Als Vernetzer können alle Verbindungen verwendet werden, die mindestens zwei ethylenisch ungesättigte Doppelbindungen oder eine ethylenisch ungesättigte Doppelbindung und eine gegenüber Säuregruppen reaktive funktionelle Gruppen tragen. Als Beispiele seien genannt: Acrylate und Methacrylate von Polyolen wie Butandioldiacrylat, Hexandioldimethacrylat, Polyglykoldiacrylat, Trimethylolpropantriacrylat oder Allylacrylat, Diallylacrylamid, Triallylamin, Diallylether, Methylenbisacrylamid oder N-Methylolacrylamid.

Als wasserlösliche Polymere können im wasserabsorbierenden Polymerisat 0 bis 30 Gew.-% teil- oder vollverseifter Polyvinylalkohol, Polyvinylpyrrolidon, Stärke oder Stärkederivate, Polyglykole oder Polyacrylsäuren enthalten sein. Das Molekulargewicht dieser Polymeren ist unkritisch, solange sie wasserlöslich sind. Bevorzugte wasserlösliche Polymeren sind Stärke, Polyvinylalkohol oder Gemische dieser Polymeren. Der bevorzugte Gehalt an solchen wasserlöslichen Polymeren im wasserabsorbierenden Polymerisat liegt bei 1 - 5 Gew.-%, insbesondere wenn Stärke und/oder Polyvinylalkohol als lösliche Polymeren vorhanden sind. Die wasserlöslichen Polymeren können als Pfropfpolymeren mit den säuregruppenenthaltenden Polymeren vorliegen.

Neben Polymerisaten, die durch vernetzende Polymerisation teilneutralisierter Acrylsäure zu erhalten sind, werden bevorzugt solche verwendet, die zusätzlich Anteile von pfropfpolymerisierter Stärke oder von Polyvinylalkohol enthalten.

Hinsichtlich der Teilchenform des eingesetzten Absorber-Polymerisates bestehen keine speziellen Einschränkungen. Das Polymere kann in Form von kugelförmigen Partikeln vorliegen, die durch inverse Suspensionspolymerisation erhalten wurden, oder in Form von unregelmäßig geformten Teilchen, die durch Trocknung und Pulverisierung der Gelmasse aus der Lösungspolymerisation erhalten werden. Die Teilchengröße liegt normalerweise im Bereich zwischen 20 und 2.000 μm, bevorzugt zwischen 50 und 850 μm.

Die zur oberflächenvernetzende Behandlung unter Reaktion der mehrfach funktionellen Gruppen einer Verbindung mit dem pulverförmigen Polymeren notwendige thermische Behandlung wird bei Temperaturen von 150 °C-250°C, bevorzugt bei 170°C - 200°C durchgeführt.

Die geeignete Temperatur ist abhängig von der Art des Behandlungsmittels und der Verweilzeit der Reaktionskomponenten bei gewählten Reaktionsbedingungen.

Beispielsweise muß die thermische Behandlung bei 150 °C über mehrere Stunden durchgeführt werden, während bei 250 °C wenige Minuten - z. B. 0,5 bis 5 Minuten - ausreichen, um die gewünschten Eigenschaften zu erzielen. Die oberflächenver-netzende thermische Behandlung kann in üblichen Trocknern oder Öfen, beispiels-weise in Drehrohröfen, Paddeltrocknern, Tellertrocknern oder in Infrarottrocknern durchgeführt werden.

Die erfindungsgemäß wiederholt durchzuführende oberflächenvernetzende Behandlung kann dabei unter denselben Bedingungen wie die erste Oberflächenvernetzung oder unter abgewandelten Bedingungen erfolgen.

Als mindestens zweifunktionelle, mit Säuregruppen reaktionsfähigen Verbindungen können demnach für die erste oberflächenvernetzende Behandlung und die erfindungsgemäße wiederholte Behandlung die gleichen oder verschiedene Verbindungen eingesetzt werden. Als derartige reaktionsfähige, oberflächenvernetzende Verbindun-gen werden Polyole, Polyamine, Alkylencarbonate allein oder in Mischung unter-einander verwendet, wobei Ethylencarbonat, Glycerin, Dimethylpropionsäure, Polyethy-lenglykol und Triethanolamin bevorzugt sind. Die zur Oberflächenvernetzung einge-setzten Verbindungen können in Form wäßriger Lösungen verwendet werden.

Die erfindungsgemäßen Mittel besitzen die Eigenschaft, daß sie große Mengen an Menstruationsblut, Urin oder andere Körperflüssigkeiten schnell absorbieren und sind daher zur Verwendung in Windeln, Damenbinden und Inkontinenzartikeln oder in Artikeln zur Wundabdeckung besonders geeignet.

Die erfindungsgemäßen, zweimal nachbehandelten Polymeren werden in Absorber-artikel für die verschiedensten Anwendungszwecke eingesetzt, z. B. durch Mischen mit Papier, Fluff oder synthetischen Fasern oder Verteilen des Mittels zwischen Substraten aus Papier, Fluff oder nichtgewebten Textilien oder durch Verformung in Trägermaterialien zu einer Bahn.

Die Absorptionsfähigkeit und Absorptionsgeschwindigkeit der erfindungsgemäßen Polymerisate unter gleichzeitig einwirkender Druckbelastung ist gegenüber den Ausgangsprodukten sehr deutlich verbessert. Da die erfindungsgemäßen Mittel die absorbierten Flüssigkeiten auch unter Druck zurückhalten, sind die Mittel besonders anwendungsfreundlich. Sie sind bevorzugt geeignet, in höheren Konzentrationen - bezogen auf hydrophiles Fasermaterial wie Fluff- als dies bisher möglich war, d.h. bei reduziertem Fluffanteil eingesetzt zu werden und zeigen ausgezeichnete Absorptionseigenschaften in Konstruktionen, die 98 - 20 Gew.-% hydrophile Fasern und 2 - 80 Gew.-%, vorzugsweise 15 - 70 Gew.-% und besonders bevorzugt 25 - 60 Gew.-% des Absorberharzes enthalten.

Die nach den beschriebenen Verfahren erhaltenen Superabsorber weisen überraschenderweise eine bedeutende Verbesserung der Aufnahme von Flüssigkeit unter Druck bei gleichzeitig hoher Gelstärke und hohen Retentionen auf, wobei insbesondere eine sehr hohe Anfangsgeschwindigkeit der Flüssigkeitsaufnahme unter Druck erreicht wird, so daß 80 % der Gesamtkapazität bereits nach 15 Minuten erreicht werden.

Die Polymeren gemäß der Erfindung besitzen bei Retentionswerten von mehr als 34 g/g eine Quellkapazität von mehr als 22 g/g für 0,9 %ige NaCl-Lösung bei einer Belastung mit 40 g/cm$^2$. Bei einer Belastung mit 60 g/cm$^2$ werden mehr als 18 g/g 0,9%ige NaCl-Lösung aufgenommen.

Besonders deutlich werden die Unterschiede zu Polymeren nach dem Stand der Technik. Bei Verdopplung der Flächenbeladung und einer Belastung mit 40 g/cm$^2$ haben die in EP 0 339 461 A beschriebenen Polymeren eine Quellkapazität von 9 g 0,9%iger NaCl-Lösung pro Gramm, während die erfindungsgemäßen Polymeren mehr als 18 g/g aufnehmen.

In einem die Praxis simulierenden Test zur Bestimmung der Saugfähigkeit von Polymeren unter Druck zeigen die erfindungsgemäßen Superabsorber eine verbesserte Saugkraft bei erhöhter Belastung.

Die erfindungsgemäßen Polymeren besitzen bei hoher Quellkapazität bzw. Retention einen deutlich höheren Quelldruck als bekannte Superabsorber.

Die erfindungsgemäßen Polymerisate sind weiterhin für die Anwendung als Wasser oder wässrige Flüssigkeiten absorbierende Komponente in strom- oder lichtleitenden Kabeln, als Komponente in Verpackungsmaterialien sowie als Bodenverbesserungs-mittel und als künstlicher Nährboden zur Pflanzenzucht geeignet.

**Testmethoden**

Zur Charakterisierung der wasserabsorbierenden Polymerisate wurden Retention (TB) und Aufnahme unter Druck (AUL) für 0,9%ige NaCl-Lösung gemessen sowie der Quelldruck bestimmt.

a) Die Retention wird nach der Teebeutelmethode und als Mittelwert aus drei Messungen angegeben. Etwa 200 mg Polymerisat werden in einen Teebeutel eingeschweißt und für 20 Minuten in 0,9%ige NaCl-Lösung getaucht. Anschließend wird der Teebeutel in einer Schleuder (23 cm Durchmesser, 1.400 Upm) 5 Minuten geschleudert und gewogen. Einen Teebeutel ohne wasserabsorbierendes Polymerisat läßt man als Blindwert mitlaufen.

$$\text{Retention} = \frac{\text{Auswaage - Blindwert}}{\text{Einwaage}} \; g/g$$

b) Die Aufnahme von 0,9%iger NaCl-Lösung unter Druck (Druckbelastung: 20, 40, 60 g/cm$^2$) wird nach der in der EP 0 339 461 A, Seite 7, beschriebenen Methode bestimmt:

In einen Zylinder mit Siebboden gibt man die Einwaage an Superabsorber und belastet das Pulver mit einem Stempel, der einen Druck von 20, 40 bzw. 60 g/cm$^2$ ausübt. Der Zylinder wird anschließend aufeinen Demand-Absorbency-Tester (DAT) gestellt, wo man den Superabsorber eine Stunde lang 0,9%ige NaCl-Lösung aufnehmen läßt. Diese Prüfung wird bei verdoppelter bzw. verdreifachter Einwaage an Superabsorber bei einem Druck von 40 g/cm$^2$ wiederholt.

c) Die Bestimmung des Quelldrucks erfolgt mit Hilfe des Stevens L. F. R. A. Texture Analyser, C. Stevens & Son Ltd, Laboratory Division, St. Albans AL1 1 Ex Hertfordshire, England.

Der zum Gerät gehörende zylindrische Meßkörper aus Glas hat eine Höhe von 3,5 cm und einen Durchmesser von 2,5 cm. Die Kreisfläche des Zylinders beträgt somit 4,91 cm$^2$.

Es werden 0,500 g Superabsorber der Fraktion 20 - 50 mesh in den dazugehörigen Meßzylinder mit 2,7 cm Durchmesser eingewogen und mit 10 ml 0,9%iger NaCl-Lösung versetzt. Danach wird der Meßzylinder mit Hilfe einer Hebebühne so weit hochgefahren, bis der Stand der Unterkante des zylindrischen Meßkörpers von der Oberfläche der sich im Meßzylinder befindlichen Probe 12 mm beträgt. Durch die Ausdehnung des Gels wird der Meßzylinder nach oben gegen eine Zwei-Weg-Kraftmeßzelle gedrückt und am Gerät in Gramm angezeigt.

d) Zur Bestimmung der Saugfähigkeit der Polymeren aus einer Matrix wird ein rundes Fluff-Pad von 6 cm Durchmesser und 2 g Gewicht liegend in einer Petrischale mit verschiedenen Mengen 0,9%ige NaCl-Lösung getränkt. In einen Plexiglaszylinder von 25,8 mm Innendurchmesser, der am Boden ein Siebgewebe hat (Maschenweite 36 µm), werden 0,20 g Polymeres eingewogen und mit einem 106 g schweren Stempel von 25 mm Durchmesser belastet. Die Zylindergruppe (Zylinder, Polymeres und Stempel) wird gewogen (A) und in die Mitte des befeuchteten Pads gestellt. Nach einer Stunde wird die Zylindergruppe zurückgewogen (B).

$$\text{Saugfähigkeit} = \frac{B - A}{0,20} \cdot \text{g/g}$$

## Beispiele

### Vergleichspolymeres A

Eine durch Lösungspolymerisation gewonnene, mit Triallylamin vernetzte Polyacrylsäure, die zu 70 Mol-% neutralisiert als Natriumsalz vorlag, wurde nach dem Trocknen und Mahlen auf 90 bis 850 µm abgesiebt und entsprechend DE 40 20 780 mit 1 Gew.-% einer 50%igen Ethylencarbonatlösung nachbehandelt. Die Kenndaten sind in Tab. 2 und 3 aufgelistet.

Tab. 2

| Retention (g/g) | Aufhahme unter Druck (AUL) | | | Quelldruck (g) |
|---|---|---|---|---|
| | 20 g/cm$^2$ (g/g) | 40 g/cm$^2$ (g/g) | 60 g/cm$^2$ (g/g) | |
| 39,5 | 28,0 | 16,0 | 9,5 | 440 |

Tab. 3

| Aufnahme in Abhängigkeit von der Flächenbelegung | | | |
|---|---|---|---|
| Flächenbelegung (mg/cm$^2$) | 31,6 | 63,2 | 94,7 |
| Aufnahme unter Druck (AUL) (g/g) bei 40 (g/cm$^2$) | 16,0 | 8,2 | 6,0 |

### Beispiel 1

Vergleichspolymeres A wird kontinuierlich mit 1000 kg/h einem Paddelmischer zugeführt und mit 1 Gew.-% einer 50%igen Ethylencarbonatlösung vermischt. Die Behandlungslösung wird mittels einer Zweistoffdüse im Mischer fein verteilt aufgegeben.

Zur thermischen Behandlung werden kontinuierlich 90 kg/h der Mischung in einen Trockner dosiert, der mit rotierenden, diskusförmigen Mischelementen ausgerüstet ist, die mit Dampf von 185 °C beheizt werden. Anschließend wird die Mischung im Wirbelbett mit Luft abgekühlt. Die Kenndaten des erhaltenen Polymeren sind in Tab. 4 und 5 aufgelistet.

Tab. 4

| Retention (g/g) | Aufnahme unter Druck (AUL) | | | Quelldruck (g) |
|---|---|---|---|---|
| | 20 g/cm$^2$ (g/g) | 40 g/cm$^2$ (g/g) | 60 g/cm$^2$ (g/g) | |
| 35,5 | 33,0 | 25,0 | 20,5 | 770 |

Tab. 5

| Aufhahme in Abhängigkeit von der Flächenbelegung | | | |
|---|---|---|---|
| Flächenbelegung (mg/cm$^2$) | 31,6 | 63,2 | 94,7 |
| Aufnahme unter Druck (AUL) (g/g) bei 40 (g/cm$^2$) | 25,0 | 20,5 | 16 |

**Beispiel 2**

1000 kg Vergleichspolymeres A werden wie in Beispiel 1 mittels einer Zweistoffdüse mit einer Lösung aus 2 kg Glycerin, 10 kg Wasser und 12 kg Ethanol kontinuierlich vermischt und in einem Silo zwischengelagert.

80 kg des oberflächenvernetzend behandelten Polymerenpulvers werden kontinuierlich stündlich in einen mit Dampfvon 180 °C beheizten, mit sichelförmigen Mischelemen-ten ausgerüsteten Paddeltrockner dosiert und nach einer mittleren Verweilzeit von ca. 30 Minuten in einer gekühlten Förderschnecke abgekühlt. Die Prüfdaten des pulverförmigen Polymeren sind in Tab. 6, 7 und 8 aufgelistet.

Tab. 6

| Retention (g/g) | Aufnahme unter Druck (AUL) | | | Quelldruck (g) |
|---|---|---|---|---|
| | 20 g/cm$^2$ (g/g) | 40 g/cm$^2$ (g/g) | 60 g/cm$^2$ (g/g) | |
| 36,5 | 32 | 24 | 19 | 680 |

Tab. 7

| Aufnahme unter Druck in Abhängigkeit von der Flächenbelegung | | | |
|---|---|---|---|
| Flächenbelegung (mg/cm$^2$) | 31,6 | 63,2 | 94,7 |
| Aufnahme unter Druck (AUL) (g/g) bei 40 (g/cm$^2$) | 24,5 | 21 | 15 |

Tab. 8

| Bestimmung der Saugfähigkeit unter Belastung aus einer Matrix | | |
|---|---|---|
| | Polymeres nach Beispiel 2 | Vergleichspolymeres A |
| Kochsalzlösung im Pad (g) | Vom Polymeren aufgesaugte Menge an Kochsalzlösung (g/g) | |
| 30 | 29,0 | 23,0 |

Tab. 8 (fortgesetzt)

| Bestimmung der Saugfähigkeit unter Belastung aus einer Matrix | | |
|---|---|---|
| | Polymeres nach Beispiel 2 | Vergleichspolymeres A |
| Kochsalzlösung im Pad (g) | Vom Polymeren aufgesaugte Menge an Kochsalzlösung (g/g) | |
| 21 | 24,5 | 16,0 |
| 15 | 21,0 | 10,5 |
| 9 | 15,0 | 10,0 |

Vergleichspolymeres B

Eine durch Lösungspolymerisation in Gegenwart von 3 Gew.-% Polyvinylalkohol (bezogen auf Acrylsäure) gewonnene, mit Trimethylolpropantriacrylat vernetzte Polyacrylsäure, die zu 70 Mol-% neutralisiert als Natriumsalz vorlag, wurde nach dem Trocknen und Mahlen auf eine Korngröße von 120 bis 500 µm abgesiebt.

Die Nachbehandlung des mit 1 Gew.-% einer 50%igen wäßrigen Ethylencarbonatlösung beschichteten Polymerpulvers erfolgt im Drehrohrofen entsprechend DE 40 20 780. Die Prüfdaten des erhaltenen pulverförmigen Polymeren sind in Tab. 9 aufgelistet.

Tab. 9

| Retention (g/g) | Aufhahme unter Druck (AUL) | | | Quelldruck (g) |
|---|---|---|---|---|
| | 20 g/cm$^2$ (g/g) | 40 g/cm$^2$ (g/g) | 60 g/cm$^2$ (g/g) | |
| 42 | 27 | 12,5 | 9,0 | 380 |

**Beispiel 3**

Das Vergleichspolymere B wird in einem hochtourig laufenden Mischer tropfenweise mit 2 Gew.-% einer Lösung aus 5 Teilen Polyethylenglykol 300, 10 Teilen Wasser und 5 Teilen Ethanol vermischt und anschließend in einem auf 180 °C vorgeheizten Drehrohrofen mit Brüdenabsaugung 60 Minuten erwärmt. Nach Abkühlung und Absiebung von 0,6 % Überkorn zeigt das pulverförmige Polymere folgende Kenndaten;

Tab. 10

| Retention (g/g) | Aufnahme unter Druck (AUL) | | | Quelldruck (g) |
|---|---|---|---|---|
| | 20 g/cm$^2$ (g/g) | 40 g/cm$^2$ (g/g) | 60 g/cm$^2$ (g/g) | |
| 36 | 30,5 | 22 | 18 | 650 |

**Patentansprüche**

1.   Pulverförmiges unlösliches, wasserquellbares, vernetztes, Wasser, wässrige oder seröse Flüssigkeiten absorbierendes Polymerisat gebildet durch Polymerisation von

a) 55 - 99,9 Gew.-% ungesättigten, polymerisierbaren, säuregruppenenthaltenden Monomeren, die zu mindestens 25 Mol% neutralisiert sind,
b) 0 - 40 Gew.-% ungesättigten, mit a) copolymerisierbaren Monomeren,
c) 0,1 - 5,0 Gew.-% eines Vernetzungsmittels und
d) 0 - 30 Gew.-% eines wasserlöslichen Polymeren,

wobei die Gewichtsmengen a) bis d) auf wasserfreies Polymer bezogen sind,
und durch Erhitzen des Polymerisatpulvers mit einer mindestens zweifunktionellen, mit Säuregruppen reaktionsfähigen Verbindung auf eine Temperatur von 150°C - 250°C unter Vernetzung der Oberfläche, dadurch gekennzeichnet, daß das Polymerisatpulver einer nochmaligen oberflächenvernetzenden Behandlung mit 0,1 - 5 Gew.-% einer mindestens zweifunktionellen, mit Säuregruppen reaktionsfähigen Verbindung bei einer Temperatur von 150°C - 250°C unterworfen worden ist.

2. Absorbierendes Polymerisat nach Anspruch 1, dadurch gekennzeichnet, daß es eine Retention von mindestens 34 g einer 0,9%igen Natriumchloridlösung pro g Harz, eine Aufnahme von mindestens 18 g, bevorzugt mindesten s 20 g einer 0,9%igen Natriumchloridlösung pro g Harz unter einem Druck von 60 g/cm

und eine Aufnahme von mindestens 12 g einer 0,9%igen Natriumchloridlösung pro g Harz bei einer Flächenbelegung von über 90 mg Harz pro cm$^2$ sowie einen Quelldruck von mindestens 600 g aufweist.

3. Absorbierendes Polymerisat nach den Ansprüchen 1 und 2, dadurch gekennzeichnet, daß es aus Acrylsäure, Methacrylsäure und/oder 2-Acrylamido-2-methyl-propansulfonsäure als säuregruppenhaltigen Monomeren gebildet ist.

4. Absorbierendes Polymerisat nach einem der Ansprüche 1 bis 3, dadurch gekennzeichnet, daß die Säuregruppen enthaltenen Monomeren zu mindestens 50 Mol% neutralisiert sind.

5. Wasserabsorbierendes Polymerisat nach einem der Ansprüche 1 bis 4, dadurch gekennzeichnet, daß es aus Acrylsäure, die zu 50 - 80 Mol% neutralisiert ist, gebildet ist.

6. Wasserabsorbierendes Polymerisat nach einem der Ansprüche 1 bis 5, dadurch gekennzeichnet, daß wasserlösliche Polymere in Konzentrationen von 1 - 5 Gew.-% eingesetzt werden.

7. Wasserabsorbierendes Polymerisat nach einem der Ansprüche 1 bis 6, dadurch gekennzeichnet, daß als wasserlösliche Polymere Stärke und/oder Polyvinylalkohol eingesetzt werden.

8. Absorbierendes Polymerisat nach einem der Ansprüche 1 bis 7, dadurch gekennzeichnet, daß die Säuregruppen des Polymeren Carboxylgruppen sind und die nochmalige oberflächenvernetzende Behandlung mit demselben vernetzenden Mittel und wie in der ersten Behandlungsstufe erfolgt.

9. Absorbierendes Polymer nach Anspruch 1 bis 7, dadurch gekennzeichnet, daß die Säuregruppen des Polymeren Carboxylgruppen sind und die nochmalige oberflächenvernetzende Behandlung mit einem zur ersten Behandlungsstufe unterschiedlichen vernetzenden Mittel oder mit Mischungen solcher Mittel erfolgt.

10. Absorbierendes Polymer nach Anspruch 1 bis 9, dadurch gekennzeichnet, daß die oberflächenvernetzenden Mittel Polyole, Polyamine, Alkylencarbonate sind.

11. Absorbierendes Polymer nach Anspruch 10, dadurch gekennzeichnet, daß die oberflächenvernetzenden Mittel Ethylencarbonat, Glycerin, Dimethylolpropionsäure, Polyethylenglykol und Triethanolamin sind und die Mittel gegebenenfalls in Form wäßriger Lösungen verwendet werden.

12. Verfahren zur Herstellung eines pulverförmigen, wasserunlöslichen, vernetzten, wäßrige und seröse Flüssigkeiten absorbierenden Polymerisates, das aus

a) 55 - 99,9 Gew.-% polymerisierten ungesättigten, polymerisierbaren, säuregruppenenthaltenden Monomeren, die mindestens zu 25 Mol% neutralisiert sind,
b) 0 -40 Gew.-% polymerisierten ungesättigten, mit a) copolymerisierbaren Monomeren,
c) 0,1 - 5,0 Gew.-% eines Vernetzungsmittels,
d) 0 - 30 Gew.-% eines wasserlöslichen Polymeren,

wobei die Gewichtsmengen a) bis d) auf wasserfreies Polymer bezogen sind, und das Polymerisatpulver mit einer mindestens zweifunktionellen, mit Säuregruppen reaktionsfähigen Verbindung aufeine Temperatur von 150 °C - 250 °C erhitzt wird, dadurch gekennzeichnet, daß das Polymerisatpulver nochmals der oberflächenver-netzenden Behandlung mit 0,1 - 5 Gew.-% einer mindestens zweifunktionellen, mit Säu-regruppen reaktionsfähigen Verbindung bei einer Temperatur von 150 °C - 250 °C unterworfen wird.

13. Verwendung von absorbierenden Polymerisaten nach den Ansprüchen 1 bis 11 als Absorptionsmittel in Körperflüssigkeiten absorbierenden Konstruktionen.

14. Verwendung nach Anspruch 13, dadurch gekennzeichnet, daß die absorbierenden Konstruktionen Sanitärartikel, vorzugsweise Windeln, Damenbinden oder Inkontinenzartikel sind.

15. Verwendung nach Anspruch 13, dadurch gekennzeichnet, daß die absorbierenden Konstruktionen Artikel zur Wundabdeckung sind.

16. Verwendung von Polymerisaten nach den Ansprüchen 1 bis 11 in Wasser und wäßrigen Flüssigkeiten absorbierenden Konstruktionen, die hydrophile Fasern und 2 bis 80 Gew.% pulverförmiges Polymerisat, bezogen auf Gesamtgewicht, enthalten.

17. Verwendung von Polymerisaten nach den Ansprüchen 1 bis 11 als Wasser oder wäßrige Flüssigkeiten absorbierende Komponente in strom- oder lichtleitenden Kabeln, als Komponente in Verpackungsmaterialien, als Bodenverbesserungsmittel und als künstlicher Boden zur Pflanzenzucht.

**Claims**

1. A powdered, insoluble, water-swellable, cross-linked polymer product which absorbs water, aqueous or serous fluids, and has been formed by polymerizing

   a) 55 - 99.9 wt.-% of unsaturated, polymerizable monomers which contain acid groups and are neutralized to at least 25 mole-%,
   b) 0 - 40 wt.-% of unsaturated monomers copolymerizable with a),
   c) 0.1 - 5.0 wt.-% of a crosslinking agent,
   d) 0 - 30 wt.-% of a water-soluble polymer,

   the weight amounts a) through d) being based on anhydrous polymer,
   and heating the polymer powder with an at least bifunctional compound reactive towards acid groups to a temperature of 150°C - 250°C to crosslink the surface, characterized in that the polymer product has been subjected to a second surface-crosslinking treatment at a temperature of 150°C - 250°C with 0.1 - 5 wt.-% of an at least bifunctional compound reactive towards acid groups.

2. The absorbing polymer of claim 1, characterized in that it has a retention of at least 34 g of a 0.9% sodium chloride solution per gram of resin, an absorption of at least 18 g, preferably at least 20 g of a 0.9% sodium chloride solution per gram of resin under a pressure of 60 g/cm$^2$, and an absorption of at least 12 g of a 0.9% sodium chloride solution per gram of resin at a surface load of more than 90 mg of resin per cm$^2$, and a swelling pressure of at least 600 g.

3. The absorbing polymer product according to claims 1 and 2, characterized in that it has been formed of acrylic acid, methacrylic acid and/or 2-acrylamido-2-methylpropanesulfonic acid as monomer containing acid groups.

4. The absorbing polymer product according to any of claims 1 to 3, characterized in that the monomers containing acid groups are neutralized to at least 50 mole-%.

5. The water-absorbing polymer product according to any of claims 1 to 4, characterized in that it has been formed of acrylic acid neutralized to 50 - 80 mole-%.

6. The water-absorbing polymer product according to any of claims 1 to 5, characterized in that water-soluble polymers at concentrations of from 1 to 5 wt.-% are used.

7. The water-absorbing polymer product according to any of claims 1 to 6, characterized in that starch and/or poly(vinyl alcohol) are used as water-soluble polymers.

8. The absorbing polymer product according to any of claims 1 to 7, characterized in that the acid groups of the polymer are carboxyl groups, and said second surface-crosslinking treatment is effected using the same crosslinking agent as in the first treatment step.

**9.** The absorbing polymer according to any of claims 1 to 7, characterized in that the acid groups of the polymer are carboxyl groups, and said second surfacecrosslinking treatment is effected using a crosslinking agent other than that of the first treatment step or mixtures of such agents.

**10.** The absorbing polymer according to any of claims 1 to 9, characterized in that the surface-crosslinking agents are polyols, polyamines, alkylene carbonates.

**11.** The absorbing polymer according to claim 10, characterized in that the surface-crosslinking agents are ethylene carbonate, glycerol, dimethylolpropionic acid, poly(ethylene glycol), and triethanolamine, and that these agents optionally are used in the form of aqueous solutions.

**12.** A process for producing a powdered, water-insoluble, crosslinked polymer product which absorbs water, aqueous or serous fluids and has been formed by polymerizing

    a) 55 - 99.9 wt.-% of unsaturated, polymerizable monomers which contain acid groups and are neutralized to at least 25 mole-%,
    b) 0 - 40 wt.-% of unsaturated monomers copolymerizable with a),
    c) 0.1 - 5.0 wt.-% of a crosslinking agent,
    d) 0 - 30 wt.-% of a water-soluble polymer,

the weight amounts a) through d) being based on anhydrous polymer,
and heating the polymer powder with an at least bifunctional compound reactive towards acid groups to a temperature of 150°C - 250°C, characterized in that the polymer product has been subjected to a second surfacecrosslinking treatment at a temperature of 150°C - 250°C with 0.1 - 5 wt.-% of an at least bifunctional compound reactive towards acid groups.

**13.** Use of the absorbing polymer products according to claims 1 to 11 as absorbents in constructions absorbing body fluids.

**14.** The use according to claim 13, characterized in that the absorbing constructions are sanitary articles, preferably diapers, liners or incontinence articles.

**15.** The use according to claim 13, characterized in that the absorbing constructions are wound dressing articles.

**16.** The use of the polymer products according to claims 1 to 11 in constructions absorbing aqueous fluids, which contain hydrophilic fibers and from 2 to 80 wt.-% of powdered polymer product, relative to the total weight.

**17.** The use of the polymer products according to claims 1 to 11 as component absorbing aqueous fluids in electroconductive or light-conducting cables, as component in packaging materials, as soil improver, and as an artificial soil in plant breeding.

**Revendications**

**1.** Polymère pulvérulent insoluble, gonflable dans l'eau, réticulé, absorbant l'eau, les liquides aqueux ou séreux, formé par la polymérisation de:

    a) de 45 à 99,9% en poids de monomères insaturés, polymérisables, contenant des groupes acides qui sont neutralisés jusqu'à au moins 25% en moles,
    b) de 0 à 40% en poids de monomères insaturés, copolymérisables avec a),
    c) de 0,1 à 5,0% en poids d'un agent de réticulation, et
    d) de 0 à 30% en poids d'un polymère soluble dans l'eau,

où les quantités pondérales a) à d) se rapportent au polymère anhydre,
et par chauffage de la poudre de polymère avec un composé réactif avec les groupes acides et au moins bifonctionnel, à une température de 150°C à 250°C, avec réticulation de la surface, caractérisé en ce que la poudre de polymère a été soumise, en outre, à un traitement supplémentaire de réticulation de la surface, avec de 0,1 à 5% en poids d'un composé réactif avec les groupes acides et au moins bifonctionnel, à une température de 150°C à 250°C.

2. Polymère absorbant selon la revendication 1, caractérisé en ce qu'il présente une rétention d'au moins 34 g d'une solution de chlorure de sodium à 0,9% par gramme de résine, une absorption d'au moins 18 g et, de préférence, d'au moins 20 g d'une solution de chlorure de sodium à 0,9% par gramme de résine sous une pression de 60 g/cm$^2$ et une absorption d'au moins 12 g d'une solution de chlorure de sodium à 0,9% par gramme de résine pour une quantité de résine appliquée sur la surface de plus de 90 mg/cm$^2$ ainsi qu'une pression de gonflement d'au moins 600 g.

3. Polymère absorbant selon les revendications 1 et 2, caractérisé en ce qu'il est formé d'acide acrylique, d'acide méthacrylique et/ou d'acide 2-acrylamido-2-méthylpropanesulfonique servant de monomère comportant des groupes acides.

4. Polymère absorbant selon l'une des revendications 1 à 3, caractérisé en ce que les monomères contenant des groupes acides sont neutralisés jusqu'à au moins 50% en moles.

5. Polymère absorbant l'eau selon l'une des revendications 1 à 4, caractérisé en ce qu'il est formé d'acide acrylique qui est neutralisé jusqu'à 50 à 80% en moles.

6. Polymère absorbant l'eau selon l'une des revendications 1 à 5, caractérisé en ce que des polymères solubles dans l'eau sont utilisés à des concentrations de 1 à 5% en poids.

7. Polymère absorbant l'eau selon l'une des revendications 1 à 6, caractérisé en ce que l'amidon et/ou l'alcool polyvinylique sont utilisés comme polymères solubles dans l'eau.

8. Polymère absorbant selon l'une des revendications 1 à 7, caractérisé en ce que les groupes acides du polymère sont des groupes carboxyle et que le traitement supplémentaire de réticulation de la surface est réalisé avec le même agent réticulant et conformément à la première étape de traitement.

9. Polymère absorbant selon les revendications 1 à 7, caractérisé en ce que les groupes acides du polymère sont des groupes carboxyle et que le traitement supplémentaire de réticulation de la surface est réalisé avec un agent réticulant différant de celui de la première étape de traitement ou avec des mélanges de tels agents.

10. Polymère absorbant selon les revendications 1 à 9, caractérisé en ce que les agents réticulants de la surface sont des polyols, des polyamines ou des carbonates d'alkylène.

11. Polymère absorbant selon la revendication 10, caractérisé en ce que les agents réticulants de la surface sont du carbonate d'éthylène, de la glycérine, de l'acide diméthylolpropionique, du polyéthylèneglycol et de la triéthanolamine et que les agents sont utilisés éventuellement sous la forme de solution aqueuse.

12. Procédé de production d'un polymère pulvérulent, insoluble, gonflable dans l'eau, réticulé, absorbant l'eau, les liquides aqueux ou séreux, formé par la polymérisation de:

a) de 55 à 99,9% en poids de monomères insaturés, polymérisables, contenant des groupes acides qui sont neutralisés jusqu'à au moins 25% en moles,
b) de 0 à 40% en poids de monomères insaturés, copolymérisables avec a),
c) de 0,1 à 5,0% en poids d'un agent de réticulation, et
d) de 0 à 30% en poids d'un polymère soluble dans l'eau,

où les quantités pondérales a) à d) se rapportent au polymère anhydre,
et par chauffage de la poudre de polymère avec un composé réactif avec les groupes acides et au moins bifonctionnel, à une température de 150°C à 250°C, caractérisé en ce que la poudre de polymère a été soumise, en outre, à un traitement supplémentaire de réticulation de la surface, avec de 0,1 à 5% en poids d'un composé réactif avec les groupes acides et au moins bifonctionnel, à une température de 150°C à 250°C.

13. Utilisation de polymères absorbants, selon les revendications 1 à 11, comme agent d'absorption dans les dispositifs absorbants les liquides physiologiques.

14. Utilisation selon la revendication 13, caractérisée en ce que les dispositifs absorbants sont des articles sanitaires et, de préférence, des langes, des serviettes hygiéniques ou des articles pour personnes incontinentes.

**15.** Utilisation selon la revendication 13, caractérisée en ce que les dispositifs absorbants sont des articles pour la couverture des blessures.

**16.** Utilisation de polymères selon les revendications 1 à 11, dans les dispositifs absorbant l'eau et les liquides aqueux, qui contiennent des fibres hydrophiles et de 2 à 80% en poids de polymère pulvérulent, rapporté au poids total.

**17.** Utilisation de polymères selon les revendications 1 à 11, comme constituant absorbant l'eau ou les liquides physiologiques dans les câbles conducteurs du courant ou de la lumière, comme constituant dans les matériaux d'emballage, comme agent d'amélioration du sol et comme sol artificiel pour la culture des plantes.